# EUROPÄISCHE PATENTSCHRIFT

(11) **EP 0 355 591 B1**
(45) Veröffentlichungstag und Bekanntmachung des Hinweises auf die Patenterteilung: **10.11.1993**
(21) Anmeldenummer: 89114769.6
(22) Anmeldetag: 09.08.1989
(51) Int. Cl.: A61M 5/165, B01D 39/16, B01D 39/08

(54) **Implantierbares Medikamenten-Dosiergerät für die Injektion eines flüssigen Medikamentes in einen lebenden Organismus**
Implantable medicament-dosing device for the injection of a liquid medicament into a living organ
Appareil implantable de dosage de médicament pour l'injection d'un médicament liquide dans un organisme vivant

(30) Priorität: 22.08.1988 DE 3828442
(43) Veröffentlichungstag der Anmeldung: 28.02.1990
(73) Patentinhaber: SIEMENS AKTIENGESELLSCHAFT, 80333 München (DE)
(72) Erfinder: Jährling, Peter, Dipl.-Ing., D-8501 Puschendorf (DE); Schweikert, Eugen, D-8526 Bubenreuth (DE)

(56) Entgegenhaltungen:
- EP-A- 0 024 601
- EP-A- 0 112 585
- DE-A- 2 116 497
- FR-A- 2 345 196
- GB-A- 1 317 015
- GB-A- 1 517 731
- US-A- 4 479 874

## Beschreibung

Die Erfindung betrifft ein implantierbares Medikamenten-Dosiergerät nach dem Oberbegriff des Patentanspruchs 1.

Bestimmte Funktionen von Komponenten des Medikamententraktes von implantierbaren Medikamenten-Dosiergeräten für flüssige Medikamente, insbesondere Pumpen, Ventile und Katheter sind empfindlich gegen Verunreinigungen des flüssigen Medikamentes in Form von festen Partikeln, die in kugel- oder auch faserförmiger Gestalt auftreten können. Aufgrund der geringen Fördervolumina solcher Systeme pro Arbeitstakt und des hydraulischen Pumpprinzips treten im Medikamententrakt Engstellen auf, deren lichte Weiten stellenweise nicht größer sind als 10 µm. An solchen Stellen können demzufolge durch Partikel dieser Größenordnung, die sich im Medikamentenstrom befinden, Verstopfungen oder Fehlfunktionen der Komponenten und/oder Leckagen des Gesamtsystems auftreten. Darüber hinaus können solche Partikel zu einer Erhöhung des Strömungswiderstandes führen, der die Pumpenleistung in unzulässiger Weise beeinflußt.

Um solche partikelabhängigen Fehlfunktionen eines implantierbaren Dosiergerätes zu vermeiden, ist es aus der US-PS 4,486,190 bereits bekannt, zwischen dem subkutanen Einstichseptum und dem mit diesem über eine Schlauchleitung verbundenen Medikamenten-Vorratsbehälter eine Filteranordnung einzufügen, die verhindert, daß die im einzufüllenden Medikament selbst vorhandenen festen Partikel in den Medikamenten-Vorratsbehälter gelangen. Durch ein an der genannten Stelle angeordnetes Filter ist es zwar möglich, herstellungsbedingte Verunreinigungen in der Medikamentenlösung selbst zu vermeiden. Nicht erfaßt mit einem solchen Filter werden jedoch Partikel, die im Medikamenten-Vorratsbehälter, etwa durch Denaturierungsprozesse des eingefüllten Medikamentes oder durch Kontamination entstehen.

Aus der DE-A-2 116 497 ist ein zweiteiliges Filter zur externen Blutfilterung bekannt, das in einem zur Förderung des Blutes dienenden Flüssigkeitstrakt vor oder hinter einer Pumpe angeordnet ist und aus einem aufstromseitigen Filtermaterial und einem abstromseitigen Auffangsieb für sich lösende Stücke des Filtermaterials besteht.

Aus der US-A-4 479 874 ist ein ebenfalls externes, mehrlagiges Filter bekannt, mit dem je nach Auswahl des Filtermaterials Porengrößen kleiner als 50 µm erreicht werden. Als in Frage kommende Filterstrukturen sind u.a. Gewebe und Spinnvliese und als Filtermaterial u.v.a. Polypropylen genannt.

Fur Oberbgriff des Ansprüches 1 wird von Gegenstand der EP-A-0 112 585 aüsgegangen, die auf der US-A-4.486.190 basiert.

Der Erfindung liegt die Aufgabe zugrunde, einen Filter so zu gestalten und anzuordnen, daß sowohl in der Medikamentenlösung herstellungsbedingt vorhandene Verunreinigungen wie solche, die sich während der Lagerungsphase des zu injizierenden Medikamentes bilden, zurückgehalten werden,ohne den Pumpvorgang, etwa durch einen zu hohen Fließwiderstand, zu beeinträchtigen.

Diese Aufgabe wird durch die im Patentanspruch angegebene Erfindung gelöst. Dadurch ist erreicht, daß alle in der Medikamentenlösung herstellungsbedingt enthaltenen oder durch deren Lagerung sich bildenden oder in diese eingetragenen Partikel unmittelbar vor dem Pumpvorgang eliminiert und dabei außerdem im Filter selbst etwa durch Faserablösung gebildete Partikel aufgefangen werden.

Anhand eines Ausführungsbeispieles wird die Erfindung im folgenden näher erläutert. Es zeigen:
- FIG 1: den prinzipiellen Aufbau des erfindungsgemäß gesteuerten implantierbaren Medikamenten-Dosiersystems und
- FIG 2: die Membrananordnung des Filters.

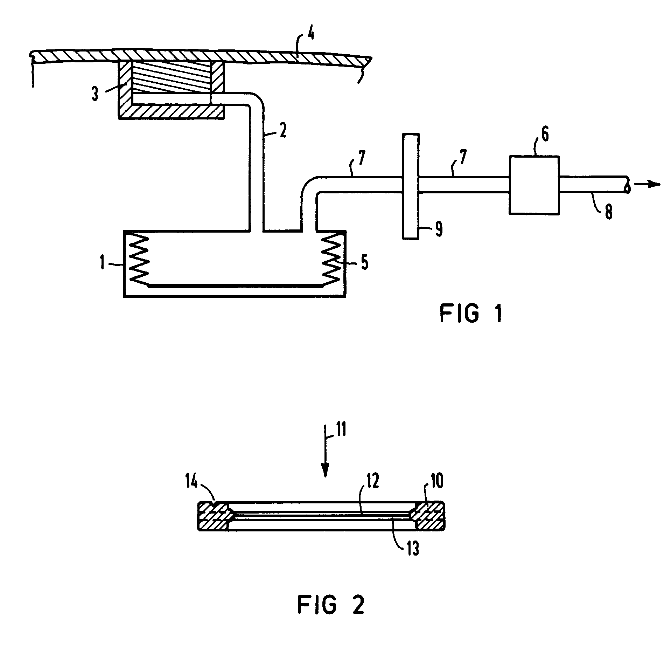

Bei dem in FIG 1 vereinfacht dargestellten prinzipiellen Aufbau eines implantierbaren Medikamenten-Dosiersystems ist ein Medikamenten-Vorratsbehälter 1 über einen Einfüllschlauch 2 mit einem sogenannten Einstichseptum 3 verbunden, welches unter der Haut 4 eines lebenden Organismus so angeordnet ist, daß ein flüssiges Medikament, beispielsweise Insulin, in bekannter Weise mit Hilfe einer Nadel in ein Membranbalgreservoir 5 des Medikamenten-Vorratsbehälters 1 nach dessen Entleerung einfüllbar ist. Eine Medikamenten-Dosierpumpe saugt das gespeicherte flüssige Medikament über einen Verbindungsschlauch 7 in dosierten Portionen ab und führt diese einem Katheter 8 zu, der die Medikamenten-Portionen an eine geeignete Stelle des Organismus transportiert und injiziert. Die Medikamenten-Dosierpumpe 6 ist mit nicht dargestellten Mitteln in der Weise ferngesteuert, daß die einzelnen Medikamenten-Portionen aufgrund einstellbarer Kriterien festlegbar sind. In den Verbindungsschlauch 7 ist eine Filteranordnung 9 eingefügt, deren Innenaufbau in FIG 2 näher dargestellt ist.

Es handelt sich dabei um ein zweilagiges Membranfilter (Sandwichfilter) mit zwei in Fließrichtung hintereinander angeordneten Membranen, die in einem ringförmigen Halterungsrahmen 10 befestigt sind. Der Pfeil 11 deutet die Fließrichtung des flüssigen Medikamentes durch das Filter an. Die in Fließrichtung erste Membran 12 besteht aus einem Faservlies aus Polypropylen mit Stützvlies und einer Orientierung gegen die Flußrichtung. Die Durchflußöffnung hat eine Flächengröße von ca. 1 cm². Die Dichte der Membran ist so gewählt, daß diese bei einem Medikamentenfluß von 50 bis 120 ml pro Minute und einer maximalen Druckdifferenz von 100 mbar eine Rückhalterate von ≧ 5 µm besitzt. Dabei liegt der sogenannte Bubbelpoint, d.h. der Druck, bei der die erste Blase durch die Membran hindurchtritt, bei ca. 10 bis 20 mbar. Die zweite, in Pfeilrichtung hinter der ersten angeordnete Membran 13 besteht aus einem Tressengewebe aus Polypropylen. Diese Webart führt zu einer Porenweite von ca. 25 µm und weist keine geraden durchgängigen Poren auf.

Beide Filtermembranen 12, 13 sind in den Halterungsrahmen 10 aus Polypropylen peripher dicht eingespritzt. Der ringförmige Halterungsrahmen 10 dient sowohl als Halterung wie auch als Dichtung gegenüber dem umgebenden Gehäuse der Filteranordnung 9. Der Halterungsrahmen 10 ist außerdem mit einer Markierung 14 für die Kenntlichmachung der Eintrittsseite des Medikamentenstromes versehen.

Alternativ oder zusätzlich zu der dargestellten und beschriebenen Anordnung des Filters zwischen dem Medikamenten-Vorratsbehälter 1 und der Medikamenten-Dosierpumpe 6 kann die Filteranordnung auch zwischen der Medikamenten-Dosierpumpe 6 und dem Katheter 8 angeordnet werden.

## Patentansprüche

1. Implantierbares Medikamenten-Dosiergerät für die Injektion eines flüssigen Medikamentes in einen lebenden Organismus mit einem Medikamententrakt, der gebildet ist aus einem subkutanen Nachfüllseptum (3) sowie einem Medikamenten-Vorratsbehälter (1), die über einen Einfüllschlauch (2) miteinander verbunden sind, einer fernsteuerbaren Medikamenten-Dosierpumpe (6), die eintrittsseitig mit dem Medikamenten-Vorratsbehälter (1) und austrittsseitig mit einem Katheter (8) verbunden ist, der das von der Medikamenten-Dosierpumpe (6) geförderte Medikament dem Organismus zuführt, sowie mit einer Filteranordnung (9) zur Filterung des flüssigen Medikamentes,
**dadurch gekennzeichnet,**
daß die Filteranordnung (9) im Medikamententrakt zwischen dem Medikamenten-Vorratsbehälter (1) und der Medikamenten-Dosierpumpe (6) und/oder zwischen der Medikamenten-Dosierpumpe (6) und dem Katheter (8) angeordnet und als wenigstens zweilagiges Membranfilter ausgebildet ist, dessen erste Membran (12) aus einem gegen die Fließrichtung des Medikamentes orientierten Polypropylen-Faservlies besteht und bei einer Flächengröße von etwa 1 cm² , einem Medikamentenfluß von 50 bis 120 ml pro Minute und einer maximalen Druckdifferenz von 100 mbar eine Rückhalterate von ≧ 5 µm besitzt und dessen zweite, stromabwärts angeordnete Membran (13) aus einem Tressengewebe aus Polypropylen ohne gerade durchgängige Poren und mit einer Porenweite von ca. 25 µm besteht.

## Claims

1. Implantable drug-metering device for injecting a liquid drug into a living organism, having a drug tract which is formed from a subcutaneous refilling septum (3) and a drug supply container (1), these being connected to one another via a flexible filling tube (2), having a remote-control drug-metering pump (6) which is connected on the inlet side to the drug supply container (1) and on the outlet side to a catheter (8) which feeds the drug conveyed from the drug-metering pump (6) to the organism, and having a filter arrangement (9) for filtering the liquid drug, characterised in that the filter arrangement (9) is arranged in the drug tract between the drug supply container (1) and the drug-metering pump (6) and/or between the drug-metering pump (6) and the catheter (8) and is constructed as an at least twin-layered membrane filter whose first membrane (12) consists of a polypropylene fibre mat oriented counter to the flow direction of the drug and, with an area of about 1 cm², a drug flow of 50 to 120 ml per minute and a maximum pressure difference of 100 mbar, has a retention rate of ≧ 5 µm, and whose second membrane (13), arranged downstream, consists of a braided fabric made of polypropylene without straight-through pores and with a pore width of about 25 µm.

## Revendications

1. Appareil implantable pour l'introduction dosée d'un médicament, servant à injecter un médicament liquide dans un organisme vivant à l'aide d'une voie d'administration du médicament, qui est formée d'un septum sous-cutané (3) pour parfaire le remplissage, ainsi que d'un réservoir (1) à médicament, qui communiquent entre eux par l'intermédiaire d'un tuyau de remplissage (2), d'une pompe (6) télécommandable d'introduction dosée du médicament, qui est raccordée côté entrée au réservoir (1) à médicament et côté sortie à un cathéter (8), qui envoie le médicament, véhiculé par la pompe (6) d'introduction dosée du médicament, dans l'organisme, ainsi que d'un dispositif de filtre (9) servant à filtrer le médicament liquide,
caractérisé par le fait
que le dispositif de filtre (9) est disposé dans la voie d'administration du médicament entre le réservoir (1) à médicament et la pompe (6) d'introduction dosée du médicament et/ou entre la pompe (6) d'introduction dosée du médicament et le cathéter (8) et est agencé sous la forme d'un filtre à membrane constitué de deux membranes, dont la première (12) est constituée d'une nappe de fibres de polypropylène orientée en sens inverse de celui de circulation du médicament, et possède, pour une surface d'environ 1 cm², un débit du médicament de 50 à 120 ml/mn et une différence maximale de pression de 100 mbar, un taux de retenue ≧ 5 µm, et dont la seconde membrane (13), disposée en aval, est constituée d'un tissu tressé de polypropylène n'ayant pas de pore traversant rectiligne et possédant des pores d'une dimension d'environ 25 µm.
